# EUROPEAN PATENT SPECIFICATION

(11) **EP 4 185 371 B1**
(45) Date of publication and mention of the grant of the patent: **09.07.2025**
(21) Application number: 21846277.8
(22) Date of filing: 07.06.2021
(51) Int. Cl.: A61N 1/36, A61N 1/05, A61N 1/375, A61N 1/372

(54) **VESTIBULAR ELECTRODE ARRAY**
VESTIBULÄRE ELEKTRODENANORDNUNG
RÉSEAU D'ÉLECTRODES VESTIBULAIRES

(30) Priority: 24.07.2020 US 202063056159 P
(43) Date of publication of application: 31.05.2023
(73) Proprietor: Cochlear Limited, Macquarie University, New South Wales 2109 (AU)
(72) Inventor: GIBSON, Peter, Macquarie University, New South Wales 2109 (AU)
(74) Representative: Grünecker Patent- und Rechtsanwälte PartG mbB
(86) International application number: PCT/IB2021/054978
(87) International publication number: WO 2022/018530

(56) References cited:
- EP-B1- 2 493 554
- WO-A1-2018/033732
- US-A- 5 645 585
- US-A1- 2015 320 550
- US-A1- 2016 199 638
- US-B1- 6 968 238
- US-B1- 8 632 577
- US-B1- 8 843 217
- US-B2- 8 372 127

## Description

### BACKGROUND

Medical devices have provided a wide range of therapeutic benefits to recipients over recent decades. Medical devices can include internal or implantable components/devices, external or wearable components/devices, or combinations thereof (e.g., a device having an external component communicating with an implantable component). Medical devices, such as traditional hearing aids, partially or fully-implantable hearing prostheses (e.g., bone conduction devices, mechanical stimulators, cochlear implants, etc.), pacemakers, defibrillators, functional electrical stimulation devices, and other medical devices, have been successful in performing lifesaving and/or lifestyle enhancement functions and/or recipient monitoring for a number of years.

The types of medical devices and the ranges of functions performed thereby have increased over the years. For example, many medical devices, sometimes referred to as "implantable medical devices," now often include one or more instruments, apparatus, sensors, processors, controllers or other functional mechanical or electrical components that are permanently or temporarily implanted in a recipient. These functional devices are typically used to diagnose, prevent, monitor, treat, or manage a disease/injury or symptom thereof, or to investigate, replace or modify the anatomy or a physiological process. Many of these functional devices utilize power and/or data received from external devices that are part of, or operate in conjunction with, implantable components.
US 8 843 217 B1 discloses an apparatus and method for triggering nerve-action potentials in each of a plurality of cochlear neurons in a cochlea of a person and in each one of a plurality of vestibular neurons in a vestibular organ of a person. A vestibular implant portion of the apparatus includes stimulation sources including a plurality of vertical-cavity-surface-emitting lasers and/or a plurality of electrodes. A transmission medium configured to deliver light signals to a vestibular neuron can include a plurality of optical fibers. The light signals can be used to optically trigger nerve-action potentials.

### SUMMARY

An apparatus according to the invention includes the features defined in claim 1.

Embodiments include the features defined in the dependent claims.

### BRIEF DESCRIPTION OF THE DRAWINGS

Embodiments of the present invention are described herein in conjunction with the accompanying drawings, in which:
FIG. 1 illustrates a view of an example vestibular stimulation system implanted relative to inner ear anatomy in accordance with certain embodiments herein.
FIG. 2 illustrates an example kit that includes the vestibular stimulator as well as other components that can be used with the implantable stimulator during implantation.
FIG. 3, which is made up of FIG. 3A, FIG. 3B, and FIG. 3C illustrates a first example implementation of a vestibular electrode array with an optical fiber bundle coupled thereto.
FIG. 3A illustrates a side view of the vestibular electrode array.
FIG. 3B illustrates a cross-section view of the vestibular electrode array taken along line B-B of FIG. 3A.
FIG. 3C illustrates a cross-section view of the vestibular electrode array taken along line C-C of FIG. 3A.
FIG. 4, which is made up of FIG. 4A, FIG. 4B, and FIG. 4C illustrates a second example implementation of a vestibular electrode array with an optical fiber bundle disposed in a lumen of the elongate carrier.
FIG. 4A illustrates a side view of the vestibular electrode array.
FIG. 4B illustrates a cross-section view of the vestibular electrode array taken along line B-B of FIG. 4A.
FIG. 4C illustrates a cross-section view of the vestibular electrode array taken along line C-C of FIG. 4A.
FIG. 5, which is made up of FIG. 5A and FIG. 5B illustrates an example method.
FIG. 6 illustrates an example view of a vestibular electrode array being advanced to a target treatment location using a stylet having a curved portion at a distal end of the stylet.
FIG. 7 illustrates an example view of a vestibular electrode array being advanced to a target treatment location using a stylet while under visualization of an optical fiber bundle.
FIG. 8 illustrates an example cochlear implant system that can benefit from use of the technologies disclosed herein.

### DETAILED DESCRIPTION

Sensory impulses relating to balance and spatial orientation are generated by the vestibular system. These sensory impulses are perceived by the brain via the vestibulocochlear nerve and provide a sense of balance and spatial orientation. But disorders affecting the vestibular system (e.g., Ménière's disease or inflammation of vestibular anatomy) can cause vestibular deficiency by interfering with these sensory impulses, thereby negatively affect one's sense of balance and spatial orientation. Vertigo can also result. Treatments for disorders of the vestibular system can include treatment with electrical stimulation via an electrode placed in the recipient's vestibule via the stapes footplate with a final position near the otolithic organs or vestibular nerve. However, placing an electrode in the proper position near or touching target anatomy (e.g., proximate the otolithic organs in the vestibule) is a surgical and technical challenge. The otolith organs, which include the saccule and utricle, are fragile and are suspended in the space of the vestibule via fine soft tissue membranes. Such treatment targets can be difficult to reach with high accuracy to ensure that electrical stimulation reaches the target structures without substantially stimulating non-target structures (e.g. ampullae of the semicircular canals). Traditional approaches for implantation include pushing an electrode array into the vestibule by a set distance or stopping when the electrode array meets a certain amount of resistance. Such blind insertion technique can be challenging to perform.

Disclosed examples include techniques and devices to facilitate implantation of a vestibular electrode array proximate a target treatment location of the recipient's vestibular system. An example vestibular electrode array can include a variety of mechanisms to provide fine control of the vestibular electrode array's position in the vestibular space. Further, the position can be guided by real time feedback mechanisms, such as fluoroscopy or electrophysiological measures to assist with positioning the vestibular electrode array.

In an example, an implanter (e.g., a clinician directly or via the use of a robotic positioner or micropositioner) controls the depth and trajectory of the vestibular stimulation array using a straight or curved stylet. The implanter can use the stylet to guide the electrodes of the vestibular electrode array to a target location (e.g., proximate the recipient's inferior vestibular nerve). The stylet can be coupled to the vestibular stimulation array, such as by being permanently embedded in the vestibular electrode array. Alternatively, the stylet is configured to be removed from a lumen in the vestibular electrode array. The coupling between the stylet and the vestibular stimulation array can be such that movement of the proximal end of the stylet controls the depth of implantation as well as an angle of the tip of the vestibular electrode array. The stylet can provide firmer control compared to a relatively malleable vestibular electrode array, such as by being relatively stiffer than the vestibular electrode array. The stylet can be configured to guide the vestibular electrode array through the oval window or a cochleostomy, such as by being sized and shaped to fit though such an opening while being coupled to the vestibular electrode array. The stylet can be a curved stylet by having a slight curve at a distal end of the stylet, which can provide fine control of the position of the tip of the vestibular electrode array by rotating the stylet. The stylet can take any of a variety of forms and can be made from any of a variety of different materials, such as one or more metals, alloys, polymers, glass, other materials, or combinations thereof. The stylet can be constructed to be stiff or malleable (e.g., relative to the vestibular electrode array). The stylet can have a shape-memory characteristic instigated by heat or electric current. The stylet and or the vestibular electrode array can include smart polymers having a shape controllable by the application of electric current. Certain implementations can be such that the stylet is configured to be mounted to the recipient's skull. For example, after the vestibular stimulation array is guided to the target treatment location with the stylet, the stylet can be mounted to the recipient's skull to resist the vestibular stimulation array being dislodged from the target treatment location.

In some examples, an endoscopic optical fiber bundle integrated with the vestibular electrode array can be used by a clinician to visualize the interior of the vestibule. The optical fibers of the optical fiber bundle can be permanently embedded in the vestibular electrode array or can be configured to reside temporarily in a lumen of the vestibular electrode array and be removable after the electrode is fixed in position. Where the optical fiber is removable from a lumen, the lumen can be configured to be closed to avoid leaving an open pathway into the vestibule after implantation. For example, in certain implementations, a clear window can be located at the tip of the vestibular electrode array so that the distal end of the lumen is not open to the vestibule (e.g., the window seals the distal end of the lumen). In addition or instead, an open lumen may be closed by partially or completely filling the lumen with a substance or closing off the proximal end of the lumen with a plug or adhesive sealant. An alternative to a lumen disposed in the vestibular electrode array for receiving the optical fiber bundle can be a groove along a side of the vestibular electrode array configured to receive the optical fiber bundle. The groove can be sealed with tissue at the entry point after implantation. The optical fiber can be held in place via an interference fit with the groove or through loops (e.g., spans over the groove) that are sufficient to keep the optical fiber bundle in place during implantation.

In some examples, the insertion of the vestibular electrode array is conducted under fluoroscopic feedback. A radiopaque component in the vestibular electrode array (e.g., metal wires, a metal stylet, or a radiopaque marker) can be visualized in the resulting fluoroscopic image. A clinician can view the fluoroscopic feedback and modify a position of the vestibular electrode array based thereon. In addition or instead, the feedback can be used with an automatic or semi-automatic robotic positioner to adjust the position of the vestibular electrode array during implantation into the vestibule. The robotic positioner can control the electrode insertion depth, angle, or other position using, for example, manipulation of a stylet (e.g., as described above) based on the fluoroscopic feedback.

In some examples, the insertion of the vestibular electrode array is conducted under real time electrophysiological feedback. For example, the electrophysiological feedback can be a response by the recipient to a stimulus. For example, the stimulus can be provided and measured by the vestibular electrode array or by one or more other devices. In some examples, the response can be a neural response or a myogenic response to the provided stimulus. The electrophysiological feedback can be used to not only monitor implantation of the vestibular electrode array with respect to vestibular anatomy. For example, in some examples, the hearing function of the recipient is monitored during insertion of the vestibular electrode array using, for example, electrocochleography. Such monitoring can be used to facilitate determining implantation's effect on the recipient's residual hearing.

During insertion, feedback regarding the positioning of the vestibular electrode array can be provided to the clinician via any of a variety of techniques, such as visual feedback or audible feedback. For example, a device can detect the position of the vestibular electrode array (e.g., the positioning of a distal tip thereof) and emit a sound with a pitch or other feature indicating the proximity of the vestibular electrode array to desirable or undesirable locations. For example, rising pitch can indicate a closer position or better response from a target nerve.

The feedback that would be provided to the clinician (or other feedback) can be provided to an automatic or semi-automatic robot in a suitable format to allow the robot to adjust the position of the electrode in the vestibule. The robot can control the electrode insertion depth, angle, or other position using a technique like the stylet invention described above.

As should be appreciated, while particular examples are illustrated and discussed herein, the disclosed vestibular stimulation prostheses and processes described herein can be integrated in any of a variety of ways in accordance with many embodiments of the invention. The discussion is not meant to suggest that the disclosed vestibular stimulation examples are only suitable for implementation within systems akin to that illustrated in and described herein. In general, additional configurations can be used to practice the methods and systems herein and/or some aspects described can be excluded without departing from the processes and systems disclosed herein.

### Vestibular Stimulation System

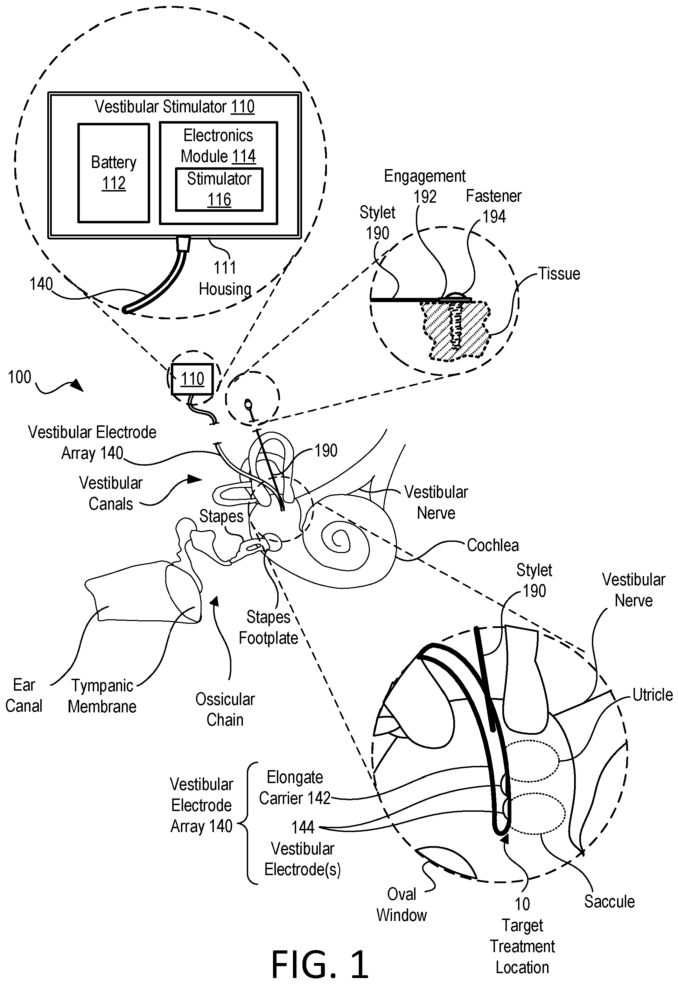

FIG. 1 illustrates a view of an example vestibular stimulation system 100 implanted relative to inner ear and vestibular anatomy of a recipient in accordance with certain embodiments herein. The vestibular stimulation system 100 is configured to provide therapeutic stimulation to a vestibular system of a recipient. In the illustrated configuration, the vestibular stimulation system 100 includes a vestibular stimulator 110 coupled to a vestibular electrode array 140 having an elongate carrier 142 with one or more vestibular electrodes 144 disposed thereon. As further illustrated, a stylet 190 has a distal end coupled to the elongate carrier 142 and a proximal end having an engagement 192 coupled to tissue of the recipient via a fastener 194.

### Vestibular Stimulation System - Vestibular Electrode Array

The vestibular electrode array 140 can refer to the combined structure that includes the elongate carrier 142 and the vestibular electrodes 144 that are disposed on the elongate carrier 142. In an example implementation, the vestibular electrode array 140 is approximately 0.4 mm in diameter and approximately 2-5mm in length with a number of vestibular electrodes 144 in the form of full-band or half-band or plate electrodes. The elongate carrier 142 is configured to dispose the one or more vestibular electrodes 144 proximate target vestibular anatomy, such as proximate the recipient's vestibular nerve, saccule, or utricle. The elongate carrier 142 can include other components, such as one or more components for connecting the elongate carrier 142 to tissue of the recipient. The elongate carrier 142 can further include one or more sensors (e.g., for sensing the vestibular system).

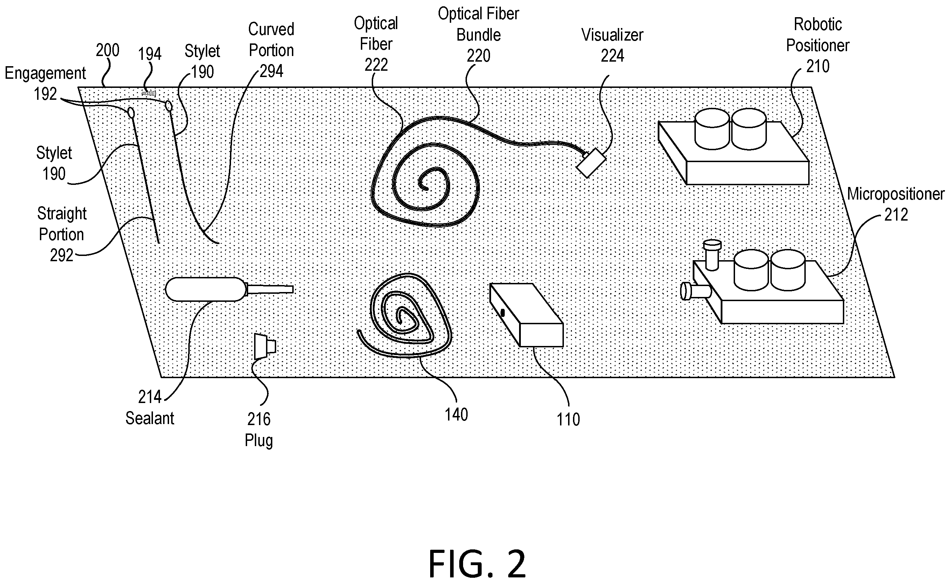

The elongate carrier 142 is a carrier for one or more components of the vestibular stimulation system 100. The elongate carrier 142 can have a first end opposite a second end. The first end can be coupled to the vestibular stimulator 110. The second end can have disposed thereon the one or more vestibular electrodes 144 and can be configured to be implanted proximate target vestibular tissue of a recipient. Thus, the elongate carrier 142 can serve as a connection between the vestibular stimulator 110 and the target anatomy. The elongate carrier 142 can further include electrical connections to connect two or more components of the vestibular stimulation system 100. For example, the elongate carrier 142 can include one or more wires that connect one or more components (e.g., the vestibular electrodes 144) with the vestibular stimulator 110. In such a configuration, the elongate carrier 142 can convey electrical stimulation signals from the vestibular stimulator 110 to the vestibular electrodes 144. The elongate carrier 142 can take any of a variety of forms. In an example, the elongate carrier 142 is made of a flexible material, such as formed with an elastomer, such as silicone. In some examples, the elongate carrier 142 can be or include an optical fiber bundle having vestibular electrodes 144 coupled thereto (see, e.g., optical fiber bundle 220 of FIG. 2, infra). The optical fibers of the elongate carrier 142 can be used with a visualizer for facilitating implantation of the vestibular electrode array 140 (see, e.g., visualizer 224 of FIG. 2, infra).

The one or more vestibular electrodes 144 are electrically conductive components via which stimulation can be provided. In some examples, the vestibular stimulation system 100 is configured to use the one or more vestibular electrodes 144 as sensors. The one or more vestibular electrodes 144 can have any of a variety of different shapes, sizes, profiles, and configurations. Example configurations of the vestibular electrodes can include configurations to encourage or resist penetrating tissue of the recipient. In some examples, the one or more vestibular electrodes 144 and the elongate carrier (e.g., a lead and body) can be as described in U.S. Patent Application No. 16/816,965, which is titled "VESTIBULAR STIMULATION PROSTHESIS", which was filed on March 12, 2020.

### Vestibular Stimulation System - Stylet

The stylet 190 can be an elongate component used to facilitate control the vestibular electrode array 140 during implantation. In many examples, the stylet 190 is stiffer than the elongate carrier 142 and imparts rigidity to the elongate carrier 142 during implantation of the vestibular electrode array 140. With the added rigidity, the implanter (e.g., the clinician or the robot performing the implantation) can manipulate the elongate carrier 142 directly or indirectly via the stylet 190.

The stylet 190 can be used to finely control a position of the vestibular electrode array 140 proximate a target vestibular treatment location 10. The stylet 190 can be manipulated by a clinician's hands directly or via a mechanical tool (e.g., the robotic positioner 210 or the micropositioner 212 of FIG. 2, infra). By manipulating a proximal end of the stylet 190, the implanter can control the depth and angle of the distal portion of the stylet 190, which in turn, controls the vestibular electrode array 140.

The stylet 190 can be constructed from any of a variety of materials. The stylet 190 can be formed from a non-bioresorbable material, a bioresorbable material, or combinations thereof. At least a portion of the stylet 190 can be formed of a bioresorbable material that dissolves on exposure to a fluid, such as a saline solution or a body fluid of the recipient. The bioresorbable material can be selected from the group consisting of polyacrylic acid (PAA), polyvinyl alcohol (PVA), polylactic acid (PLA) and polyglycolic acid (PGA). The non-bioresorbable material can be, for example, a biocompatible metal, alloy, polymer, or glass material. In some examples, the stylet 190 can include, be, or be a part of an optical fiber bundle. Various stylets 190 and materials for use in embodiments disclosed herein, including those described in US 6,421,569; US 7,269,461; US 7,555,352; US 7,894,916; and US 9,427,568.

In some examples, one or both of the stylet 190 and the vestibular electrode array 140 can be formed at least partially from a shape memory material. For example, a bimetallic element (e.g., nickel/titanium) can be shaped to take a substantially straight configuration at a first temperature (e.g., room temperature) and bend into another shape once it is exposed to a second temperature (e.g., body temperature during or after implantation). In examples, the shape memory material can be an electroactive polymer having shape memory characteristics that are modifiable via the application of an electric current. For example, the material can be held in a first position during insertion by an application of electric current to the material and then the material can be allowed to relax to a second position with the ceasing of the application of the electric current.

The stylet 190 can be configured to be stiff or malleable. In some examples, the stylet 190 can have different regions with different stiffnesses. For example, in some implementations, a portion of the stylet 190 being disposed within or proximate the elongate carrier 142 can be relatively more malleable than a remainder of the stylet 190.

In some examples, the stylet 190 can be partially or wholly removed after implantation of the vestibular electrode array 140. In some examples, the stylet 190 can partially or wholly remain implanted in the recipient after implantation is complete. For example, in the illustrated example, the stylet 190 can hold the vestibular electrodes 144 proximate a target vestibular treatment location 10 after implantation. The stylet 190 can have a distal end coupled to the elongate carrier 142 and a proximal end having an engagement 192 coupled to tissue of the recipient via a fastener 194. The engagement 192 can be the portion of the stylet configured to facilitate coupling with tissue of the recipient, and the fastener 194 can be the component that couples with the tissue of the recipient. For example, the engagement 192 can be an eyelet formed at the proximal end of the stylet 190 and the fastener 194 can be a bone screw configured to be driven through the eyelet and into the recipient's skull with a head of the bone screw interacting with the eyelet to resist movement of the stylet 190.

### Vestibular Stimulation System - Vestibular Stimulator

The vestibular stimulator 110 can be a component of the vestibular stimulation system 100 that generates the stimulation signals that are to be applied to the vestibular system. The vestibular stimulator 110 can include a housing 111 in which one or more components of the vestibular stimulator 110 are disposed. The vestibular stimulator 110 can include any of a variety of components, such as a battery 112, an electronics module 114, and a stimulator 116 disposed within a housing 111.

The housing 111 can be an encasement constructed from or coated in a biocompatible material to facilitate long-term implantation of the vestibular stimulator 110 in a recipient. The housing 111 can surround and hermetically seal one or more components of the vestibular stimulator. In examples, the housing 111 includes a header providing an interconnection between one or more components within and external to the housing 111.

The electronics module 114 can include one or more components that provide vestibular stimulation or other functionality. In some examples, the electronics module 114 includes one or more components for receiving a signal (e.g., from external or implanted devices and sensors) and one or more components for converting the signal into a stimulation signal, such as a transceiver and an antenna. In some examples, the electronics module 114 generates a stimulation signal without regard to a signal from an external device. In some examples, the stimulation signal is generated according to a predetermined stimulation schedule that defines when and at what intensity the stimulation is to be applied, and the electronics module can include one or more components defining the stimulation schedule.

In some examples, the electronics module 114 includes one or more processors (e.g., central processing units or microcontrollers) coupled to memory components (e.g., flash memory or ROM) storing instructions that, when executed, cause performance of an operation described herein. In some examples, the electronics module 114 generates and monitors parameters associated with generating and delivering the stimulus (e.g., output voltage, output current, or line impedance). In some examples, the electronics module 114 can generate a telemetry signal that includes telemetry data based on one or more of the parameters. The electronics module 114 can send the telemetry signal to an external device or store the telemetry signal in memory for later use or retrieval.

As illustrated, the electronics module 114 can include a stimulator 116. The stimulator 116 generates electrical stimulation signals for use in stimulating tissue. The stimulator 116 can use stimulation control signals generated by the electronics module 114 (e.g., based on a stimulation schedule, based on one or more sensors, or based on commands received from an external device) to generate electrical stimulation signals (e.g., electrical current signals) for delivery to the recipient's vestibular anatomy via the one or more vestibular electrodes 144. In this way, the vestibular stimulation system 100 electrically stimulates the recipient's vestibular anatomy (e.g., nerve cells thereof), in a manner that causes the recipient to perceive vestibular percepts. In some examples, in addition to or instead of causing the recipient to perceive vestibular percepts, the stimulation can have a calming, masking, or impeding effect on an over-active vestibular system of the recipient. The stimulation can be monopolar stimulation or multipolar stimulation (e.g., bipolar).

The battery 112 can be a component configured to store power. The battery 112 can include, for example, one or more rechargeable or non-rechargeable batteries. In some examples, the vestibular stimulator 110 can be configured to receive power from another device, such as an external device or another implanted device. The power stored by the battery 112 can be distributed to the other components of the vestibular stimulator 110 as needed for operation.

The vestibular stimulator 110 can be a standalone vestibular stimulation prosthesis. In other examples, the vestibular stimulation system 100 can be part of another implanted medical device to add vestibular stimulation capabilities to the device. For instance, the implanted medical device can be a sensory prosthesis relating to one or more of the recipient's senses. For example, the sensory prosthesis can be a prosthesis relating to one or more of the five traditional senses (vision, hearing, touch, taste, and smell) and/or one or more of the additional senses. The sensory prosthesis can be an auditory prosthesis medical device configured to treat a hearing-impairment of the recipient. Where the sensory prosthesis is an auditory prosthesis, the sensory prosthesis can take a variety of forms including a cochlear implant, an electroacoustic device, a percutaneous bone conduction device, a passive transcutaneous bone conduction device, an active transcutaneous bone conduction device, a middle ear device, a totally-implantable auditory device, a mostly-implantable auditory device, an auditory brainstem implant device, a hearing aid, a tooth-anchored hearing device, a personal sound amplification product, other auditory prostheses, and combinations of the foregoing (e.g., binaural systems that include a prosthesis for a first ear of a recipient and a prosthesis of a same or different type for the second ear). In examples, the sensory prosthesis can be or include features relating to bionic eyes. Technology disclosed herein can also be relevant to applications with devices and systems used in for example, sleep apnea management, tinnitus management, and seizure therapy. Technology disclosed herein can be used with sensory devices such as consumer auditory devices (e.g., a hearing aid or a personal sound amplification product).

In some examples, the vestibular stimulator 110 (and the vestibular stimulation system 100 as a whole) can include one or more aspects of the devices, methods, and computer programs for generating one or more signals for the electrical stimulation of the saccule and utricle of a patient as described in WO 2017/081335.

The vestibular stimulator 110 can be implemented in any of a variety of ways. In some implementations, the vestibular stimulator 110 can lack electronics and instead include an inductively or capacitively coupled wire. In some examples, power can be harvested from the body through heat or movement.

### Vestibular Stimulation System - Vestibular Anatomy

FIG. 1 further shows the vestibular stimulation system 100 disposed in relation to vestibular and auditory anatomy. Among the anatomy shown, is the ear canal, which is part of the auditory anatomy. Disposed across an end of ear canal is a tympanic membrane which vibrates in response to sound waves and is vibrationally coupled to the oval window (also known as the fenestra ovalis), which is adjacent round window, through the ossicular chain. The ossicular chain includes the malleus, the incus, and the stapes. In typical anatomy, the stapes includes the stapes footplate (also known as the base). The ossicular chain causes the oval window to vibrate in response to the vibration of the tympanic membrane, which causes motion within the cochlea that causes nerve impulses to be generated and transferred through the auditory nerve to the brain where they are perceived as sound.

In addition to the auditory anatomy, vestibular anatomy is also shown: the vestibular canals (also known as the semicircular canals) and the otolith organs. The vestibular canals are three canals (known as the horizontal canal, the superior canal, and the posterior canal) that allow rotational movement to be sensed. The otolith organs, which include the utricle and saccule, allow linear movement to be sensed. Rotational and linear movement cause appropriate nerve impulses to be generated via the vestibular anatomy and transferred through the vestibular nerve to the brain where they are perceived as motion.

The human skull is formed from a number of different bones that support various anatomical features. These bones are omitted from the figure to aid the viewer. The temporal bone is situated at the side and base of the recipient's skull. The temporal bone is covered by a portion of the recipient's skin, muscle, and fat, which can collectively be referred to as tissue. The temporal bone can be referred to as having a superior portion and a mastoid portion. The superior portion comprises the section of the temporal bone that extends superior to the auricle. That is, the superior portion is the section of the temporal bone that forms the side surface of the skull. The mastoid portion is positioned inferior to the superior portion and is the section of the temporal bone that surrounds the middle ear.

The various components of the vestibular stimulation system 100 can be disposed with reference to this anatomy. In particular, the illustrated configuration shows the elongate carrier 142 (and thus the one or more vestibular electrodes 144 coupled thereto) having a distal tip disposed at a target vestibular treatment location 10 proximate the saccule of a recipient's left or right auditory anatomy. In the illustrated example, the target vestibular treatment location 10 is such that the vestibular electrodes 144 are in contact with the vestibule without piercing the vestibule. In other examples, the target vestibular treatment location 10 is within the vestibule and the elongate carrier 142 at least partially pierces the vestibule and contacts underlying tissue. In an example, the vestibular electrodes 144 are disposed within the vestibule proximate one or both of the saccule and the utricle. In addition, while the vestibular stimulator 110 and the proximal end of the stylet 190 are illustrated as being located superior to the cochlea, the vestibular stimulator 110 and the stylet 190 can be located in any of a variety of locations.

One or more components of the vestibular stimulation system 100 can be provided to a clinician can be provided as part of a kit. The kit can further include components to facilitate implantation. An example of the kit is described in relation to FIG. 2.

### Example Kit

FIG. 2 illustrates an example kit 200 that includes the vestibular stimulator 110 as well as other components that can be used with the vestibular stimulator 110 during implantation. The kit 200 can be a packaging of one or more components for use with the vestibular stimulator 110. The kit 200 can include components for use during implantation. The illustrated kit 200 includes the vestibular stimulator 110, the vestibular electrode array 140, a first stylet 190 having a straight portion 292, a second stylet 190 having a curved portion 294, a fastener 194, a robotic positioner 210, a micropositioner 212, a sealant 214, a plug 216, an optical fiber bundle 220 of one or more optical fibers 222 coupled to a visualizer 224. More or fewer components can be included as part of the kit 200. In addition, one or more of the components can be provided as part of another kit.

The first stylet 190 can be a stylet 190 having a straight portion 292 proximate the distal tip of the stylet 190. The second stylet 190 can be a stylet 190 having a curved portion 294 proximate the distal tip of the stylet 190. The curve in the stylet 190 can be used to permit a user to more finely control position of the tip of the electrode by rotating the stylet 190. As illustrated, the stylets 190 are separate from the vestibular electrode array 140. In some examples, one or more of the stylets 190 are removably or irremovably pre-installed into the vestibular electrode array 140. For example, a distal portion of the stylet 190 is embedded in the vestibular electrode array 140. A distal portion of a stylet 190 can be coupled to the vestibular electrode array 140 via a dissolvable adhesive that is dissolved during implantation so the stylet 190 can be removed when it is no longer used. In some examples, the kit 200 includes a plurality of different stylets 190 of various characteristics (e.g., stiffnesses) from which a clinician can choose.

The fastener 194 can be a component configured to secure the stylet 190. For example, in the illustrated embodiment, the fastener 194 is a bone screw configured to secure a proximal portion of the stylet 190 to the recipient's skull to secure the stylet 190. The fastener 194 can take other forms. The fastener 194 can be an adhesive, a tack, or a component to encourage tissue growth. As further illustrated, the fastener 194 is a component separate from the stylet 190 and is configured to interface with an engagement 192 of the stylet. In other examples, the fastener 194 is at least partially integrated with the stylet 190.

The robotic positioner 210 can be a component configured to automatically perform at least one aspect of guiding the vestibular electrode array 140 to the target vestibular treatment location 10. In some examples, the robotic positioner 210 can be configured to automatically perform all of the guidance of the vestibular electrode array 140 to the target location or the robotic positioner 210 can automatically assist a clinician in performing certain implantation functions. The robotic positioner 210 can include one or more sensors, processors, and actuators configured to obtain data regarding the position of the vestibular electrode array 140, process the data, and activate the one or more actuators based thereon. The vestibular electrode array 140, stylet 190, or another component can be coupled to the robotic positioner 210 (e.g., to one or more of the actuators thereof) such that actuation of one or more of the actuators causes movement of the vestibular electrode array 140.

The micropositioner 212 can be a manually-manipulatable device that permits a clinician to finely control the position of the vestibular electrode array 140. For example, the micropositioner 212 can permit a user to more finely control a position of the vestibular electrode array 140 than the user would be able to without the micropositioner 212. The vestibular electrode array 140, the stylet 190, or another component can be coupled to the micropositioner 212 such that manipulation of the micropositioner 212 causes movement of the vestibular electrode array 140. In some examples, the micropositioner 212 can be or include a universal joint that can be used to manipulate the stylet 190 and lock the stylet 190 in place as needed.

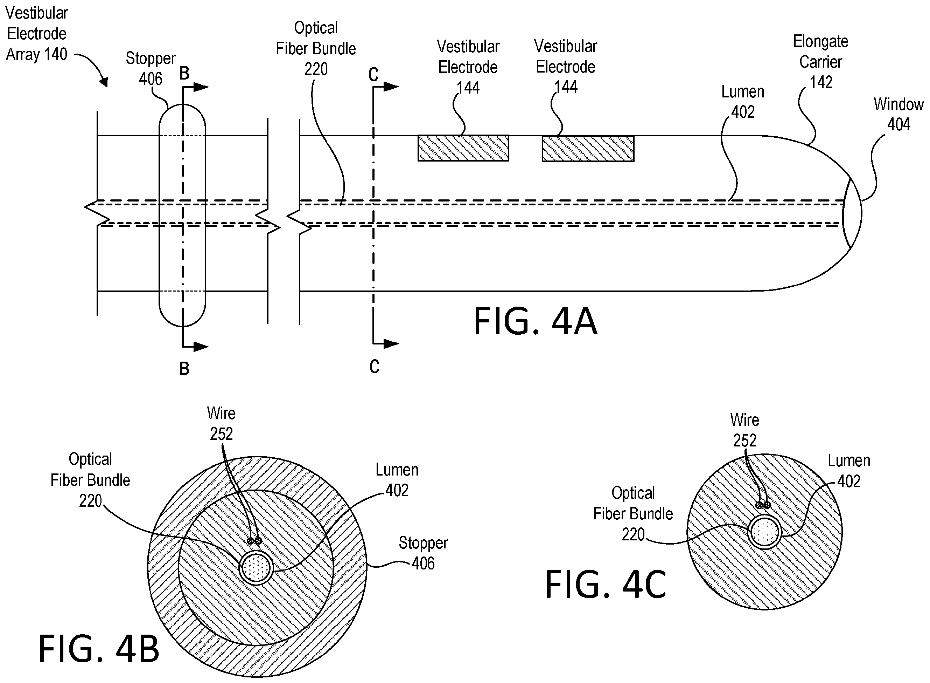

The sealant 214 and plug 216 can be components configured to seal or plug an opening in a component of the kit 200. For example, as described in more detail below in FIG. 4, the vestibular electrode array 140 can include a lumen for carrying the optical fiber bundle 220. The sealant 214 or plug 216 can be configured to seal an opening of the lumen of the elongate carrier. In many examples, the sealant 214 is a paste contained in a tube that, when applied and allowed to cure or dry, forms a durable barrier. The plug 216 can be a pre-formed object that can be applied to an opening to form a barrier.

The optical fiber bundle 220 of one or more optical fibers 222 can be components to facilitate the transmission of light. For example, the optical fiber bundle 220 can be used to transmit light to a region proximate the distal end of the vestibular electrode array 140 during implantation and carry reflected light back to permit a clinician to visualize implantation and facilitate navigation of the vestibular electrode array 140 to a desired treatment location. The proximal end of the optical fiber bundle 220 can be coupled to visualizer 224. In some examples, the optical fiber bundle 220 is configured to be operated separate from the vestibular electrode array 140 (e.g., the optical fiber bundle 220 need not be coupled to the vestibular electrode array 140). In other examples, the elongate carrier 142 can be configured to receive or couple with the optical fiber bundle 220 such that movement of the elongate carrier 142 causes movement of the optical fiber bundle 220.

The visualizer 224 can be a component configured to couple with a proximal end of the optical fiber bundle 220 to facilitate use of the optical fiber bundle 220 during implantation of the vestibular electrode array 140. The visualizer 224 can be or include a light source, an eyepiece, a camera lens, and/or an image sensor. Where the visualizer 224 includes the light source, the visualizer 224 receives light output by the light source and directs the light through one or more of the optical fibers 222. The light can then exit the distal end of the optical fibers 222 to provide illumination during implantation. Where the visualizer 224 includes an eyepiece or camera lens, such components can receive light reflected through one or more of the optical fibers 222 from an object illuminated by the light emitting from the distal end of the optic fibers 222. A magnifying or focusing device can be further incorporated with the visualizer 224. The camera lens can be part of a video system that permits recording of the image detected by an image sensor. The video can be used in real time during implantation, such as by being displayed on a screen.

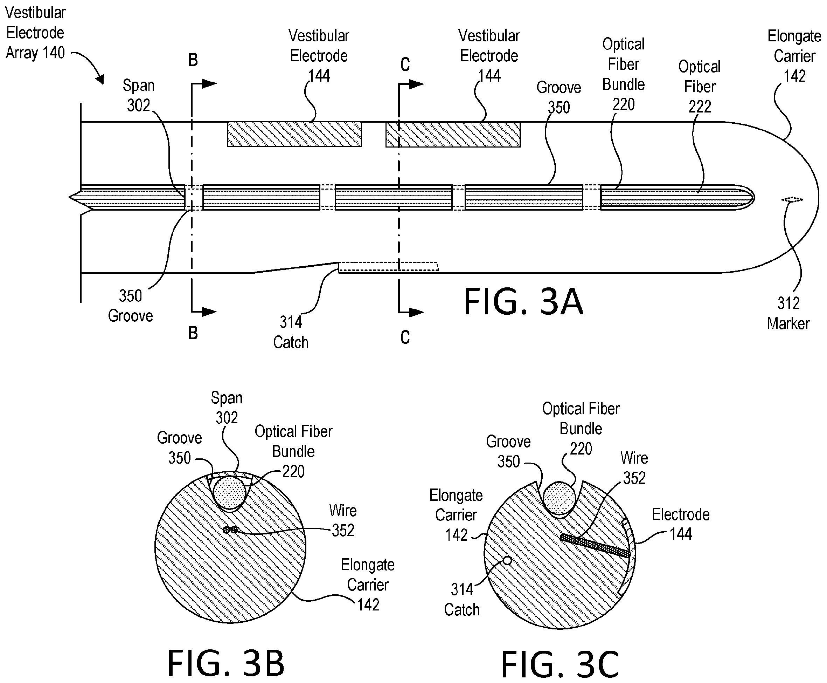

The optical fiber bundle 220 or other components can be configured to couple with the vestibular electrode array 140. The coupling can be achieved in any of a variety of ways. An example implementation is shown in FIG. 3.

### First Example Vestibular Electrode Array with Optical Fiber

FIG. 3A, FIG. 3B, and FIG. 3C make up FIG. 3, which illustrates a first example implementation of a vestibular electrode array 140 with an optical fiber bundle 220 coupled thereto. FIG. 3A illustrates a side view of the vestibular electrode array 140. FIG. 3B illustrates a cross-section view of the vestibular electrode array 140 taken along line B-B of FIG. 3A. FIG. 3C illustrates a cross-section view of the vestibular electrode array 140 taken along line C-C of FIG. 3A.

As illustrated, the vestibular electrode array 140 includes an elongate carrier 142 that is configured to be implanted proximate a vestibular organ of a recipient. The illustrated vestibular electrode array 140 includes at least one vestibular electrode 144 coupled to the elongate carrier 142. As further illustrated, an optical fiber bundle 220 of one or more optical fibers 222 is coupled to the elongate carrier 142 such that implantation of the elongate carrier 142 can be monitored via the optical fiber bundle 220. In addition, the illustrated elongate carrier 142 defines a groove 350 at least partially along a side of the elongate carrier 142.

The groove 350 can be configured to receive the optical fiber bundle 220. In an example, the groove 350 can be sized and shaped to permit the optical fiber bundle 220 to be disposed in the groove 350 for a distance without the optical fiber bundle 220 extending past a maximum diameter of the vestibular electrode array 140. In another example, at least a portion of the optical fiber bundle 220 can extend past the maximum diameter of the elongate carrier 142.

The elongate carrier 142 can be configured to retain the optical fiber bundle 220 in the groove 350. In some examples, the elongate carrier 142 can be so configured by defining the groove 350 such that an interference fit exists between the groove 350 and the optical fiber bundle 220. In the illustrated example, the elongate carrier 142 comprises one or more spans 302 over the groove 350. The one or more spans 302 can be configured to retain the optical fiber bundle 220 in the groove 350). A span 302 can be a portion of material extending over the groove 350 and defining an opening underneath the span 302 through which the optical fiber bundle 220 can pass. In the illustrated example, the spans 302 extend completely over the groove 350 in certain sections along the elongate carrier 142. In other examples, the spans 302 can be cantilevered over the groove 350 such that the spans 302 partially overhang the groove 350 to retain the optical fiber bundle 220 in the groove 350 but that can be bent to permit the optical fiber bundle 220 to be inserted into or removed from the groove 350. The fit between the optical fiber bundle 220 and the opening formed by the groove 350 and the spans 302 can retain the optical fiber bundle 220 in place during implantation such that the optical fiber bundle 220 can be used to visualize implantation.

As shown in FIGS. 3B and 3C, the illustrated vestibular electrode array 140 includes one or more wires 352. The distal ends of the wires 352 can be coupled to a vestibular electrode 144 and the proximal ends of the wires 352 can couple to the vestibular stimulator 110, such that stimulation signals can be transmitted along the wires 352 to cause stimulation via the vestibular electrodes 144. Other signals can be transmitted, such as sensing signals.

The illustrated vestibular electrode array 140 further includes a catch 314. The catch 314 can be a feature of the elongate carrier 142 with which the stylet 190 can interact. For example, the catch 314 can be configured to receive a stylet 190 to guide the elongate carrier 142 during insertion of the elongate carrier 142. The catch 314 can be so configured by, for example, being sized and shaped to receive the stylet within the catch 314. In some examples the stylet 190 is configured to be irremovably coupled with the catch 314 (e.g., the stylet 190 cannot be removed without causing damage to the stylet 190 or the elongate carrier 142). For example, the stylet 190 can be glued into the catch 314 or embedded within the material forming the catch 314 during manufacture of the elongate carrier 142. In other examples, the stylet 190 can be configured to be removable from the catch 314 (e.g., the stylet 190 can be removed without causing substantial damage to the stylet 190 or the elongate carrier 142). For example, there can be a threaded connection between the stylet 190 and the catch 314 such that the stylet 190 can be removed by unscrewing the stylet 190 from the catch 314. In other examples, the stylet 190 can be glued into the catch 314 with a dissolvable adhesive. The stylet 190 can be removed via the application of a solution to dissolve the adhesive.

One or more of the components of the vestibular electrode array 140 can be radiopaque to facilitate visualization of the vestibular electrode array 140. In the illustrated example, the vestibular electrode array 140 further includes a marker 312. The marker 312 can be radiopaque.

As mentioned above, the implementation shown in FIG. 3 is an example. Another example implementation is shown in FIG. 4.

### Second Example Vestibular Electrode Array with Optical Fiber

FIG. 4A, FIG. 4B, and FIG. 4C make up FIG. 4, which illustrates a second example implementation of a vestibular electrode array 140 with an optical fiber bundle 220 disposed in a lumen 402 of the elongate carrier 142. FIG. 4A illustrates a side view of the vestibular electrode array 140. FIG. 4B illustrates a cross-section view of the vestibular electrode array 140 taken along line B-B of FIG. 4A. FIG. 4C illustrates a cross-section view of the vestibular electrode array 140 taken along line C-C of FIG. 4A.

The lumen 402 can extend at least partially along the length of the elongate carrier 142 and be sized and shaped to accommodate the optical fiber bundle 220 being disposed therein. The optical fiber bundle 220 can be removably or irremovably disposed therein. A distal portion of the lumen 402 can terminate at a window 404. The window 404 can be an optically transparent region. The optical fiber bundle 220 can have a distal end disposed proximate the window 404 such that at least some of the light transmitted via the optical fiber bundle 220 can pass through the window to permit visualization of an area proximate the window. In some examples, the window 404 hermetically seals the distal end of the lumen 402. In some examples, the window 404 is a discrete component from the elongate carrier 142. In other examples, the window 404 can be a portion of the elongate carrier 142 that is optically transparent. In some examples, the window 404 is more optically transparent than the elongate carrier 142. In some examples, the window 404 can be configured as a lens.

As further illustrated, the vestibular electrode array 140 can include a stopper 406. The stopper 406 can be a component coupled to the elongate carrier 142 at a location and being configured to resist the elongate carrier 142 being advanced more than a predetermined distance. For example, the stopper 406 can be formed as a buildup of material. In the illustrated example, the stopper 406 is an annular collar disposed around a location of the elongate carrier 142. In an example, the stopper 406 can be sized such that the stopper 406 cannot extend through a particular portion of a recipient's anatomy, such as the recipient's oval window, the recipient's round window, a cochleostomy opening, or an opening in the recipient's stapes. The stopper 406 can be coupled with the elongate carrier 142 (e.g., through a friction fit) such that if movement of the stopper 406 is blocked, then movement of the elongate carrier 142 is resisted. Thus, the stopper 406 can resist or prevent the elongate carrier 142 from extending further than a predetermined distance. In some examples, the stopper 406 is a separate component from the elongate carrier and can be disposed in a configurable position along the elongate carrier 142.

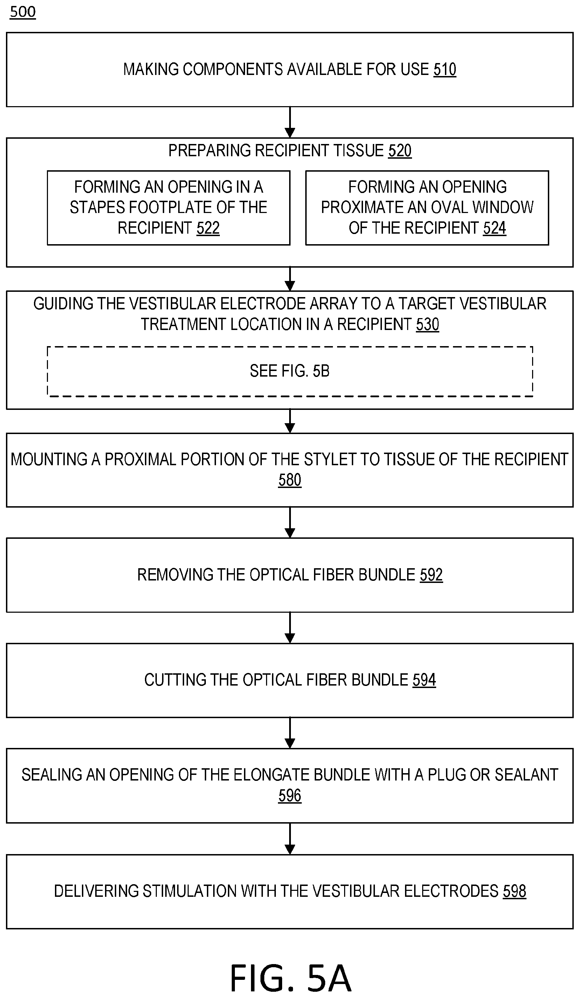

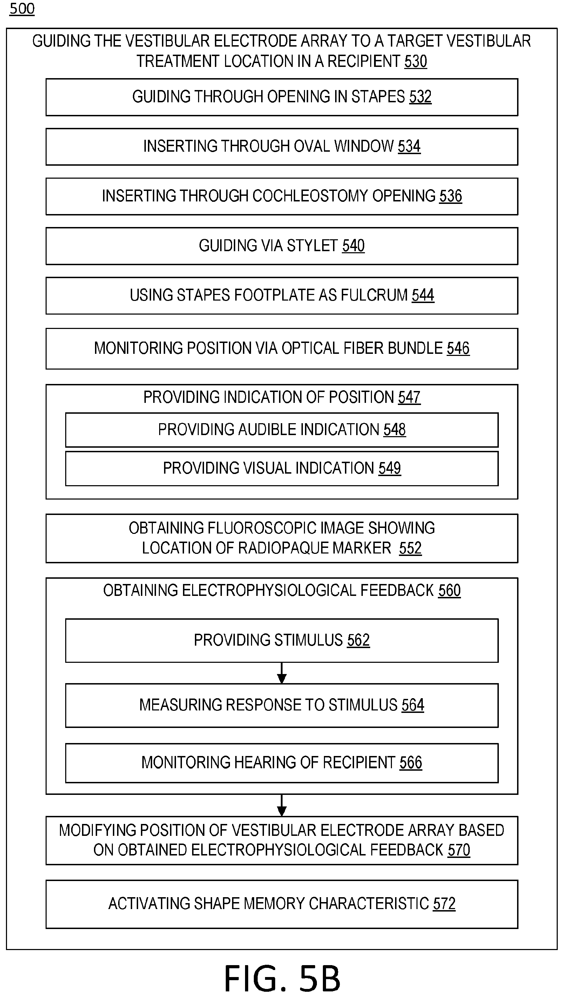

The components of the vestibular electrode array of FIG. 4 and the other figures described above can be used in any of a variety of ways. An example method that can use one or more of the components described above is shown in FIG. 5.

### Example Method

FIG. 5A and FIG. 5B make up FIG. 5, which illustrates an example method 500 for implanting a vestibular electrode array and stimulating with the array. The method 500 can include operation 510.

Operation 510 includes making components available for use. For example, the operation 510 can include making a vestibular electrode array 140 available for use. In some examples, the operation 510 can include making a vestibular electrode array 140 available for use that has a stylet 190 pre-installed. In some examples, the operation 510 can include making a vestibular electrode array 140 available for use that has an optical fiber bundle 220 pre-installed. Making the vestibular electrode array 140 available for use can include making a vestibular electrode array 140 available for use that has an elongate carrier 142 constructed from an optical fiber bundle 220 and having vestibular electrodes 144 coupled to the optical fiber bundle 220. The operation 510 can include making a stylet 190 having a straight portion 292 proximate a distal end of the stylet 190 available for use. For example, the operation 510 can include making a stylet 190 having a curved portion 294 proximate a distal end of the stylet 190 available for use. In some examples, the operation 510 can include installing or coupling one or more components with respect to each other. For example, the operation 510 can include coupling the stylet 190 with the vestibular electrode array 140 (e.g., by coupling the stylet 190 with the catch 314). In an example, the stylet 190 is removably inserted into the catch 314 of the vestibular electrode array 140. The operation 510 can include coupling the optical fiber bundle 220 with the vestibular electrode array 140, such as by placing the optical fiber bundle 220 at least partially within the groove 350 (e.g., and securing the optical fiber bundle 220 with the spans 302) or the lumen 402 depending on the configuration of the vestibular electrode array 140. Following operation 510, the flow of the method 500 can move to operation 520.

Operation 520 can include preparing recipient tissue. For example, the operation 520 can include sterilizing tissue of the recipient and surgically accessing an implantation area in the recipient. For example, the clinician can form one or more incisions in tissue proximate a location where the vestibular stimulation system 100 is to be implanted and remove tissue to expose an implantation area. The opening formed by one or more incisions can be sized and shaped to allow for the performance of operations described herein to be performed through the opening. In some examples, surgically accessing the implantation area includes performing a mastoidectomy or a cochleostomy. The operation can further include forming or enlarging a posterior tympanotomy, which can include exposing the oval window, such as by superiorly enlarging the posterior tympanotomy. In some examples, surgically accessing the implantation area includes identifying the target vestibular treatment location 10. In some examples, a pocket or a bone bed can be formed for the vestibular stimulator 110 to be disposed in. Operation 520 can include operation 522 and 524. Operation 522 includes forming an opening in a stapes of the recipient. For example, the opening can be formed in a footplate of the stapes, such as by using a surgical drill. Operation 524 includes forming an opening proximate the oval window of a recipient. In an example, the operation 524 includes removing the stapes of the recipient and forming the opening in the oval window. In another example, an opening is formed through the anulus of the oval window beside the stapes footplate. Following operation 520, the flow of the method 500 can move to operation 530.

Operation 530 includes guiding the vestibular electrode array 140 to a target vestibular treatment location 10 in the recipient. The guiding can be performed in whole in or part by a human clinician. In some examples, the guiding can be performed in whole or in part via a robotic positioner 210 or a micropositioner 212, such as by moving the vestibular electrode array with the robotic positioner 210 or the micropositioner 212. As shown in more detail in FIG. 5B, operation 530 can include a variety of operations.

Operation 532 can include guiding a component through an opening in the stapes footplate. As described above, the method 500 can include forming an opening in the stapes footplate of the recipient. A portion of the vestibular electrode array 140 can be guided through the opening in the stapes. In some examples, additional components are also guided through the opening, such as the stylet 190 and the optical fiber bundle 220.

Operation 534 can include guiding a component through an opening in the oval window. As described above, the method can include forming an opening in the recipient's oval window. A portion of the vestibular electrode array 140 can be guided through the opening in the oval window. In some examples, additional components are also guided through the opening, such as the stylet 190 and the optical fiber bundle 220.

Operation 536 can include guiding a component through a cochleostomy opening. As described above, the method can include performing a cochleostomy. A portion of the vestibular electrode array 140 can be guided through the opening formed by the cochleostomy. In some examples, additional components are also guided through the opening, such as the stylet 190 and the optical fiber bundle 220.

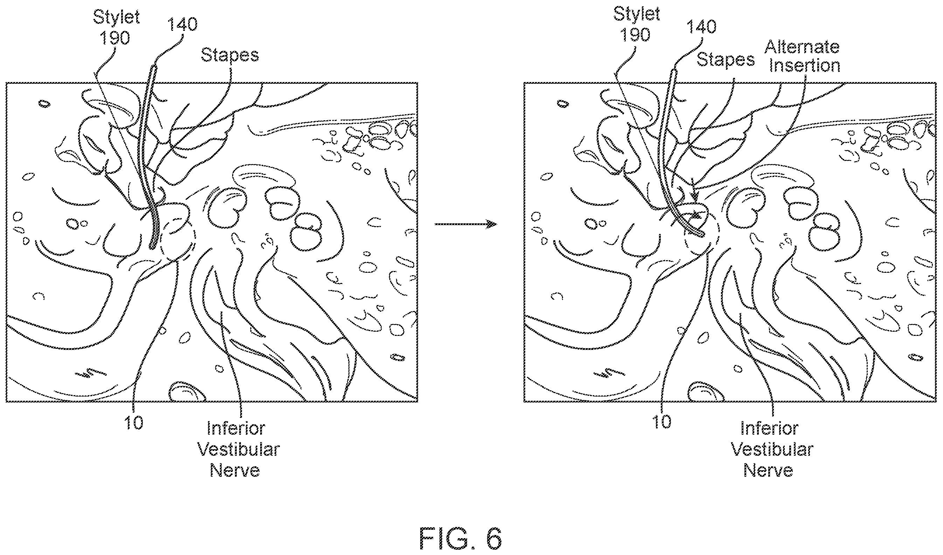

Operation 540 can include guiding via a stylet 190. For example, guiding the vestibular electrode array 140 to the target vestibular treatment location 10 in the recipient can include moving a proximal end of the stylet 190 (e.g., advancing, retracting, or tilting the proximal end) to control a depth of the vestibular electrode array 140 and an angle of a tip of the vestibular electrode array 140. In some examples, the operation 540 can include twisting the stylet 190 substantially along the stylet's long axis. Twisting the curved stylet 190 can cause rotation of the distal tip of the stylet 190, which can induce movement in a plane substantially perpendicular to the long axis. That movement can provide fine control of the position of the tip of the vestibular electrode array 140. An example of twisting a curved stylet 190 is shown in FIG. 6.

FIG. 6 illustrates an example view of a vestibular electrode array 140 being advanced to a target vestibular treatment location 10 using a stylet 190 having a curved portion at a distal end of the stylet. As illustrated, rotation of the stylet 190 causes the distal portion of the vestibular electrode array 140 to rotate into position at the target vestibular treatment location 10. An alternate insertion location through a cochleostomy opening is further illustrated as an arrow.

Returning to FIG. 5B, operation 544 can include using a stapes footplate as a fulcrum. For example, the footplate of the stapes can be used as a fulcrum to permit fine control of a distal portion of the stylet 190 while a user manipulates a proximal portion of the stylet 190. A relatively proximal portion of the stylet 190 can be placed against the footplate of the stapes and the point of contact can act as a pivot point by which to manipulate a more distal portion of the stylet 190.

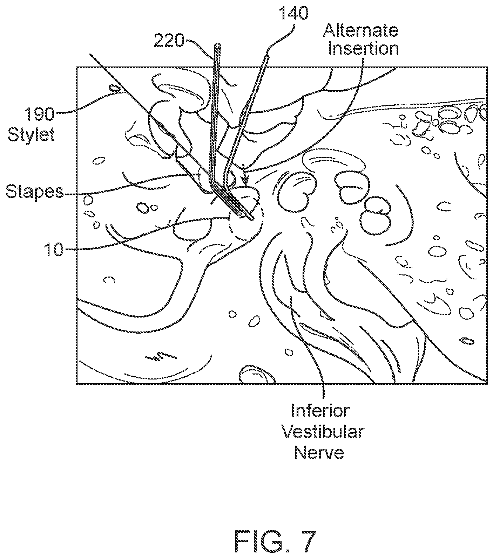

Operation 546 can include monitoring a position via an optical fiber bundle 220 of one or more optical fibers 222. The position of the distal portion of the vestibular electrode array 140 can be monitored. In some examples, the position of the vestibular electrodes 144 can be monitored. The monitoring can be the result of direct visualization of the component (e.g., as may be accomplished by an optical fiber bundle 220 separate from the vestibular electrode array 140) or via indirect visualization. For example, the distal tip of the optical fiber bundle 220 can be proximate the distal end of the vestibular electrode array 140 such that little or no portion of the vestibular electrode array 140 is visible. Nonetheless, the position of the vestibular electrode array 140 or a component thereof can be inferred by what is visualized through the optical fiber bundle 220. For example, the implanter can assume that what is being visualized through the distal portion of the optical fiber bundle 220 sufficiently closely corresponds to the position of the distal portion of the vestibular electrode array 140 that the visualization can be useful in guiding the vestibular electrode array 140. The monitoring with the optical fiber bundle 220 can include using the optical fiber bundle 220 to carry light generated proximate the proximal end of the optical fiber bundle 220 out the distal end of the optical fiber bundle 220. The light can illuminate the area proximate the distal end of the optical fiber bundle 220 and can be reflected back and carried through the optical fiber bundle 220 to reach a visualizer 224 coupled to the proximal end of the optical fiber bundle 220. The implanter can visualize the reflected light directly via the visualizer 224 or via another component connected to the visualizer 224. An example illustration showing the monitoring of a position via the optical fiber bundle 220 is shown in FIG. 7.

FIG. 7 illustrates an example view of a vestibular electrode array 140 being advanced to a target vestibular treatment location 10 using a stylet 190 while under visualization of an optical fiber bundle 220. As illustrated, the optical fiber bundle 220 is coupled to the vestibular electrode array 140 as the vestibular electrode array is advanced to the target vestibular treatment location 10 with the stylet 190. The vestibular electrode array 140 is shown as being advanced through an opening in the recipient's stapes. But an alternate insertion location through a cochleostomy opening is further illustrated as an arrow.

Returning to FIG. 5B, operation 547 can include providing an indication of position. For example, the position can be the position of a portion of the vestibular electrode array 140, a component thereof, or another component (e.g., a portion of the stylus 190 or the optical fiber bundle 220). A device can detect the position of the component using any of a variety of techniques (e.g., surgical navigation position determining techniques). The indication of position can be provided via any of a variety of techniques, such as visual feedback or audible feedback. In some examples, the operation 547 can include operation 548 or operation 549. Operation 548 can include providing an audible indication of position. For example, the operation can include providing the audible indication of the position via a speaker. The relative position can be indicated via a change in pitch of an audible indication. Operation 549 can include providing a visual indication of position. In some examples, the visual indication is provided on a display of a computer system that tracks the position. A surgeon can use a surgical microscope for visualizing anatomy. The view from the surgical microscope can be provided at the display of a computer system and one or more items of feedback can be provided on the display (e.g., concurrent with the view from the surgical microscope as a heads up display). In some examples, one or more lights be used to indicate position (e.g., by flashing or changing color).

Operation 552 can include obtaining a fluoroscopic image showing a location of a component of the vestibular electrode array 140. For example, the vestibular electrode array 140 can include a radiopaque marker 312. The obtained fluoroscopic image can show a location of the radiopaque marker 312 or another radiopaque component of the vestibular electrode array 140 relative to the target vestibular treatment location 10. The implanter can use the obtained image to determine a position of the vestibular electrode array 140 relative to the target vestibular treatment location 10, which can be used to guide the vestibular electrode array 140.

Operation 560 can include obtaining electrophysiological feedback. The electrophysiological feedback can include direct or indirect feedback regarding a position of the vestibular electrode array 140. The feedback can further include information regarding an effect of the implantation on the recipient, such as an effect of the implantation on the recipient's residual hearing. Operation 560 can include operation 562, operation 564, and operation 566.

Operation 562 can include providing a stimulus. For example, providing the stimulus can include providing the stimulus with at least one vestibular electrode 144 of the vestibular electrode array or the effect of the implantation procedure on the recipient. Operation 564 can include measuring a response to the stimulus. For example, measuring the response can include measuring a neural or myogenic response to the provided stimulus. The response can be measured using one or more of a variety of different sensors. In some examples, the vestibular electrode array 140 includes a sensor. In some examples, one or more of the vestibular electrodes 144 can act as a sensor. The one or more sensors can generate data indicative of the recipient's response. For example, where the response is a neural response, the one or more sensors can detect electrical activity indicative of the response to the stimulation. Such a neural response can detect whether the vestibular electrodes 144 are able to stimulate the recipient's vestibular nerve or other tissue. Where the response is a myogenic response, one or more sensors within the recipient or external to the recipient can detect muscle movement of the recipient that can be in response to the applied stimulation.

Operation 566 can include monitoring hearing of the recipient. For example, the hearing or other aspects of the recipient can be monitored using electrocochleography. Electrocochleography can include measuring compound action potential of the auditory nerve in response to test signals applied, using, for example, an electrode disposed proximate the eardrum or the middle ear of a patient. In another example, an auditory brainstem response measurement device may be employed to measure the electrical potential near the region of the brain (e.g. the dorsal and ventral cochlear nuclei of the brainstem) that processes the cochlear input response to test signals applied to an implantable transducer. In this example, one or more electrodes positioned at various locations on the scalp of a patient may be employed to measure the auditory brainstem response potentials. Examples of techniques for performing electrocochleography are described in US 2005/0131272 and US 10,413,728.

Operation 570 can include modifying a position of the vestibular electrode array 140 based on obtained electrophysiological feedback. The feedback (e.g., measured myogenic or neural response) can be used as an indication of whether the vestibular electrode array 140 is disposed in the correct location such that the vestibular electrodes 144 can stimulate the desired tissue. For example, the vestibular electrode array 140 can be advanced to a location believed to be appropriate, stimulation can be provided, and, based on the response, the vestibular electrode array 140 can remain in the location or be moved to a different location to retest until the response has desired characteristics. The obtained electrophysiological feedback can indicate that the vestibular electrode array 140 is in an incorrect position, and the implanter can modify the position of the vestibular electrode array 140 to attempt to move the vestibular electrode array 140 to the correct location.

Operation 572 can include activating a shape memory characteristic. For example, the operation 572 can include permitting activation of the shape memory characteristic caused by a body temperature of the recipient. The operation 572 can include applying heat to activate the shape memory characteristic. In some examples, the operation 572 can include activating the shape memory characteristic with the application of electrical current. The activation of the shape memory characteristic can cause the component having the shape memory characteristic (e.g., the vestibular electrode array 140 or the stylet 190) to change shape. In some examples, the change shape can facilitate implantation. For instance, the shape memory characteristic can cause the vestibular electrode array 140 to be relatively straight and stiff during the application of an electrical current, which can facilitate passage of the vestibular electrode array 140 through an opening. After the vestibular electrode array 140 passes through the opening, the electrical current can be stopped, which can cause the vestibular electrode array 140 to relax.

As described above, the operation 530 of guiding the vestibular electrode array to a target vestibular treatment location 10 can include any of a variety of operations. Following operation 530, the flow of the process can move to operation 580, which is shown in FIG. 5A.

Operation 580 can include mounting a proximal portion of the stylet 190 to tissue of the recipient. In an example, operation 580 can be performed before or after guiding the vestibular electrode array 140 to the target vestibular treatment location 10. For example, the tissue of the recipient to which the proximal portion of the stylet 190 is mounted can be the recipient's skull. This mounting can fix a position of the proximal portion of the stylet 190, which can resist movement of the distal portion of the stylet 190. The mounting technique can vary depending on the configuration of the stylet 190, engagement 192, and the fastener 194. For example, where the fastener 194 is a screw, the fastener 194 can be screwed into the recipient's skull such that the fastener 194 engages with the engagement 192 (e.g., an eyelet through which the screw extends). Where the fastener 194 is an adhesive, the operation 580 can include adhering the engagement 192 (e.g., which can be a structure, such as a paddle, configured to provide a surface area to which the adhesive can adhere). The stylet 190 can be left behind in the recipient after implantation and the recipient has healed.

Operation 592 can include removing the optical fiber bundle 220. For example, the operation 592 can include removing the optical fiber bundle 220 from the elongate carrier 142. The optical fiber bundle 220 can be removed from the groove 350. In other examples, the optical fiber bundle 220 can be removed from the lumen 402. The optical fiber bundle 220 can be removed via the application of force, such as by pulling on the optical fiber bundle 220 in a manner sufficient to overcome a friction or other fit. In some examples, a shape memory characteristic or dissolvable material can be used to remove the optical fiber bundle 220. For example, applying or ceasing application of an electrical current to a shape memory component can cause the optical fiber bundle 220 to be released.

Operation 594 can include cutting the optical fiber bundle 220. In some examples, after implanting the vestibular electrode array 140, the optical fiber bundle 220 is no longer of use. Rather than removing the optical fiber bundle 220 (e.g., from the lumen 402), the optical fiber bundle 220 can be left in the recipient (e.g., the recipient can be sutured to close the one or more incisions with the optical fiber bundle 220 remaining in the recipient), but a portion of the optical fiber bundle 220 extending outside of the elongate carrier 142 can cut and removed. The portion of the optical fiber bundle 220 remaining in the elongate carrier 142 can be allowed to remain in the recipient. In some examples, the portion remaining can be treated with an adhesive or sealant after the cutting. The portion of the optical fiber bundle 220 remaining in the vestibular electrode array 140 can be left behind in the recipient after implantation and the recipient has healed.

Operation 596 can include sealing an opening of the elongate bundle with a plug 216 or sealant 214. For example, during some implantation procedures, an opening can be created or left in the elongate carrier (e.g., by the removal of the optical fiber bundle 220 or by cutting the optical fiber bundle 220). The opening can be sealed via application of a plug 216 or sealant 214 to close the opening.

Operation 598 can include delivering stimulation with the vestibular electrodes. For example, the vestibular stimulator 110 can cause the vestibular electrodes 144 to deliver stimulation to tissue proximate the vestibular electrodes 144. The stimulation can be delivered to the target vestibular treatment location 10. In some examples, the stimulation is provided continuously or periodically. In some examples, the stimulation is provided based on output from one or more sensors (e.g., accelerometers or gyroscopes that measure the recipient's balance).

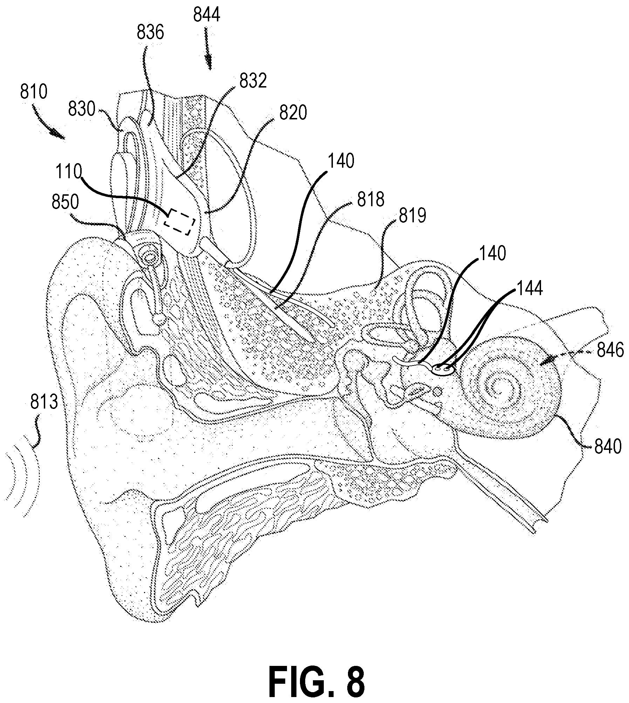

The above techniques are described primarily in the context of a standalone vestibular stimulator 110. But, in other examples, the vestibular stimulation system 100 can be part of another implanted medical device to add vestibular stimulation capabilities to the device. For instance, the implanted medical device can be a sensory prosthesis relating to one or more of the recipient's senses, such as the cochlear implant system described below in FIG. 8.

### Cochlear Implant System

FIG. 8 illustrates an example cochlear implant system 810 that can benefit from use of the technologies disclosed herein. The cochlear implant system 810 includes an implantable component 844 typically having an internal receiver/transceiver unit 832, a stimulator unit 820, and an elongate lead 818. The internal receiver/transceiver unit 832 permits the cochlear implant system 810 to receive signals from and/or transmit signals to an external device 850. The external device 850 can be a button sound processor worn on the head that includes a receiver/transceiver coil 830 and sound processing components. Alternatively, the external device 850 can be just a transmitter/transceiver coil in communication with a behind-the-ear device that includes the sound processing components and microphone.

The implantable component 844 includes an internal coil 836, and preferably, a magnet (not shown) fixed relative to the internal coil 836. The magnet can be embedded in a pliable silicone or other biocompatible encapsulant, along with the internal coil 836. Signals sent generally correspond to external sound 813. The internal receiver/transceiver unit 832 and the stimulator unit 820 are hermetically sealed within a biocompatible housing, sometimes collectively referred to as a stimulator/receiver unit. Included magnets (not shown) can facilitate the operational alignment of an external coil 830 and the internal coil 836, enabling the internal coil 836 to receive power and stimulation data from the external coil 830. The external coil 830 is contained within an external portion. The elongate lead 818 has a proximal end connected to the stimulator unit 820, and a distal end 846 implanted in a cochlea 840 of the recipient. The elongate lead 818 extends from stimulator unit 820 to the cochlea 840 through a mastoid bone 819 of the recipient. The elongate lead 818 is used to provide electrical stimulation to the cochlea 840 based on the stimulation data. The stimulation data can be created based on the external sound 813 using the sound processing components and based on the sensory prosthesis settings. As illustrated, the stimulator unit 820 further includes the stimulator 116 configured to deliver stimulation to vestibular tissue of the recipient via electrodes of the vestibular electrode array 140 disposed proximate the oval window of the recipient. The vestibular electrode array 140 connects the stimulator 116 to the vestibular electrodes 144 of the vestibular electrode array 140.

In certain examples, the external coil 830 transmits electrical signals (e.g., power and stimulation data) to the internal coil 836 via a radio frequency (RF) link. The internal coil 836 is typically a wire antenna coil having multiple turns of electrically insulated single-strand or multi-strand platinum or gold wire. The electrical insulation of the internal coil 836 can be provided by a flexible silicone molding. Various types of energy transfer, such as infrared (IR), electromagnetic, capacitive and inductive transfer, can be used to transfer the power and/or data from external device to cochlear implant. While the above description has described internal and external coils being formed from insulated wire, in many cases, the internal and/or external coils can be implemented via electrically conductive traces.

Other sensory prostheses can benefit from technologies described herein. For example, the technology disclosed herein can be implemented with a direct acoustic stimulator prosthesis configured to generate vibrations and conduct the vibrations to move perilymph in scala tympani to activate hair cells to cause hearing percepts. Such a stimulator can include an actuator, a stapes prosthesis and a coupling element connecting the actuator to the stapes prosthesis. In an example, the prosthesis stimulation arrangement can be implanted and/or configured such that a portion of stapes prosthesis abuts a recipient's round or oval window. In examples, the portion of the prosthesis that abuts the oval window can include one or more vestibular electrodes 144 described herein for stimulating vestibular anatomy.

As should be appreciated, while particular uses of the technology have been illustrated and discussed above, the disclosed technology can be used with a variety of devices in accordance with many examples of the technology. The above discussion is not meant to suggest that the disclosed technology is only suitable for implementation within systems akin to that illustrated in the figures. For examples, while certain technologies described herein were primarily described in the context of auditory prostheses (e.g., cochlear implants), technologies disclosed herein are applicable to medical devices generally (e.g., medical devices providing pain management functionality or therapeutic electrical stimulation, such as deep brain stimulation). In general, additional configurations can be used to practice the processes and systems herein and/or some aspects described can be excluded without departing from the processes and systems disclosed herein. Further, the techniques described herein can be applicable to determining a recipient's response to other stimuli, such as visual stimuli, tactile stimuli, olfactory stimuli, taste stimuli, or other stimuli. Likewise, the devices used herein need not be limited to auditory prostheses and can be other medical devices configured to support a human sense, such as bionic eyes.

This disclosure described some aspects of the present technology with reference to the accompanying drawings, in which only some of the possible aspects were shown. Other aspects can, however, be embodied in many different forms and should not be construed as limited to the aspects set forth herein. Rather, these aspects were provided so that this disclosure was thorough and complete and fully conveyed the scope of the possible aspects to those skilled in the art.

As should be appreciated, the various aspects (e.g., portions, components, etc.) described with respect to the figures herein are not intended to limit the systems and processes to the particular aspects described. Accordingly, additional configurations can be used to practice the methods and systems herein and/or some aspects described can be excluded without departing from the methods and systems disclosed herein.

Similarly, where steps of a process are disclosed, those steps are described for purposes of illustrating the present methods and systems and are not intended to limit the disclosure to a particular sequence of steps. For example, the steps can be performed in differing order, two or more steps can be performed concurrently, additional steps can be performed, and disclosed steps can be excluded without departing from the present disclosure. Further, the disclosed processes can be repeated.

Although specific aspects were described herein, the scope of the technology is not limited to those specific aspects. One skilled in the art will recognize other aspects or improvements that are within the scope of the present technology. Therefore, the specific structure, acts, or media are disclosed only as illustrative aspects. The scope of the technology is defined by the following claims.

## Claims

1. An apparatus comprising:
an elongate carrier (142) configured to be implanted proximate a vestibular organ of a recipient;
at least one vestibular electrode (144) coupled to the elongate carrier (142); and
an optical fiber bundle (220) of one or more optical fibers (222),
**characterized in that**
the optical fiber bundle (220) is coupled to the elongate carrier (142) such that implantation of the elongate carrier (142) can be monitored via the optical fiber bundle (220); and
the optical fiber bundle (220) is removable from the elongate carrier (142).

2. The apparatus of claim 1, wherein the elongate carrier (142) defines a groove (350) at least partially along a side of the elongate carrier (142).

3. The apparatus of claim 2, wherein the elongate carrier (142) comprises one or more spans (302) over the groove (350); and
wherein the one or more spans (302) are configured to retain the optical fiber bundle (220) in the groove (350).

4. The apparatus of any of claims 1, 2, or 3, wherein one or more components of the apparatus are radiopaque.

5. The apparatus of any of claims 1, 2, or 3, wherein the elongate carrier (142) defines a catch (314) configured to receive a stylet (190) to guide the elongate carrier (142) during insertion of the elongate carrier (142).

6. A kit comprising:
an implantable vestibular stimulator (110) having a biocompatible housing (111); and
the apparatus according to claim 5, wherein the elongate carrier (142) is configured to be coupled to or is coupled to the implantable vestibular stimulator (110);
wherein the stylet is any one or both of: a first stylet (190) having a straight portion proximate a first stylet distal end of the first stylet; and a second stylet (190) having a curved portion proximate a second stylet distal end of the second stylet.

7. The kit of claim 6, further comprising: a fastener (194) configured to secure the first stylet (190) or the second stylet (190) to a recipient's skull.

8. The kit of any of claims 6 or 7, further comprising:
a stopper (406) coupled to the elongate carrier (142) at a location configured to resist the elongate carrier (142) being advanced more than a predetermined distance.

9. The kit of any of claims 6, 7, or 8,
wherein the kit (200) includes both the first stylet (190) and the second stylet (190); wherein the kit (200) further includes a robotic positioner (210);
wherein the kit (200) further includes a micropositioner (212);
wherein the kit (200) further includes a sealant (214) or plug (216) configured to seal an opening of a lumen (402) of the elongate carrier (142);
wherein the first stylet (190) or the second stylet (190) is constructed from a metal, a glass, or a polymer;
wherein the first stylet (190) or the second stylet (190) is removable from a catch (314) of the elongate carrier (142);
wherein the first stylet (190) or the second stylet (190) is stiff;
wherein the first stylet (190) or the second stylet (190) is malleable;
wherein the first stylet (190), the second stylet (190) or the elongate carrier (142) has shape memory characteristics;
wherein the first stylet (190), the second stylet (190), or the elongate carrier (142) has shape memory characteristics configured to be activated by temperature;
wherein the first stylet (190), the second stylet (190), or the elongate carrier (142) has shape memory characteristics configured to be activated by electric current; or
wherein the first stylet (190), the second stylet (190), or the elongate carrier (142) includes a polymer having shape memory characteristics configured to be activated by electric current.

10. The kit of any one of the claims 6-9, wherein the kit is configured for guiding, via the stylet, the vestibular electrode array (140) to the target vestibular treatment location (10) in the recipient using (544) a stapes footplate of the recipient as a fulcrum to permit fine control of a distal portion of the stylet (190).

11. The kit of claim 10,
wherein the guiding (530) includes:
obtaining (560) electrophysiological feedback regarding a position of the vestibular electrode array (140); and
modifying the position of the vestibular electrode array based on the obtained electrophysiological feedback.

12. The kit of claim 11, wherein guiding (530) the vestibular electrode array (140) to the target vestibular treatment location (10) in the recipient further includes:
obtaining (552) a fluoroscopic image showing a location of a radiopaque marker (312) of the vestibular electrode array (140) relative to the target vestibular treatment location (10).

13. The kit of claim 11 or 12, wherein obtaining the electrophysiological feedback includes:
providing (562) a stimulus using at least one vestibular electrode (144) of the vestibular electrode array (140); and measuring (564) a neural or myogenic response to the provided stimulus.

14. The kit of any of claims 11 - 13, further comprising means for:
monitoring (566) hearing of the recipient using electrocochleography, and/or
providing (547) a visual indication or an audible indication of the position, and/or
providing (548) the audible indication of the position via a change in pitch of an audio tone.

15. The kit of any of claims 11 -14,
wherein the guiding (530) is performed at least in part by a robotic positioner (210).

## Patentansprüche

1. Vorrichtung, die umfasst:
einen länglichen Träger (142), der zum Implantieren in der Nähe eines vestibulären Organs eines Empfängers ausgeführt ist;
wenigstens eine vestibuläre Elektrode (144), die mit dem länglichen Träger (142) gekoppelt ist; sowie
ein Lichtleitfaser-Bündel (220) aus einer oder mehreren Lichtleitfaser/n (222),
**dadurch gekennzeichnet, dass**
das Lichtleitfaser-Bündel (220) so mit dem länglichen Träger (142) gekoppelt ist, dass Implantation des länglichen Trägers (142) über das Lichtleitfaser-Bündel (220) überwacht werden kann; und
das Lichtleitfaser-Bündel (220) von dem länglichen Träger (142) gelöst werden kann.

2. Vorrichtung nach Anspruch 1, wobei der längliche Träger (142) eine Nut (350) wenigstens teilweise entlang einer Seite des länglichen Trägers (142) aufweist.

3. Vorrichtung nach Anspruch 2, wobei der längliche Träger (142) ein oder mehrere Joch/e (302) über der Nut (350) umfasst; und
wobei das eine oder die mehreren Joch/e (302) so ausgeführt ist/sind, dass sie das Lichtleitfaser-Bündel (220) in der Nut (350) hält/halten.

4. Vorrichtung nach einem der Ansprüche 1, 2 oder 3, wobei ein oder mehrere Komponente/n der Vorrichtung strahlenundurchlässig ist/sind.

5. Vorrichtung nach einem der Ansprüche 1, 2 oder 3, wobei der längliche Träger (142) einen Anschlag (314) aufweist, der zum Aufnehmen einer Sonde (190) ausgeführt ist, mit der der längliche Träger (142) bei Einführung des länglichen Trägers (142) geführt wird.

6. Bausatz, der umfasst:
einen implantierbaren vestibulären Stimulator (110) mit einem biokompatiblen Gehäuse (111); sowie
die Vorrichtung nach Anspruch 5, wobei der längliche Träger (142) zum Koppeln mit dem implantierbaren vestibulären Stimulator (110) ausgeführt oder mit ihm gekoppelt ist;
wobei die Sonde eine erste Sonde (190) mit einem geraden Abschnitt nahe an einem ersten distalen Sonden-Ende der ersten Sonde oder/und eine zweite Sonde (190) mit einem gekrümmten Abschnitt nahe an einem zweiten distalen Sonden-Ende der zweiten Sonde ist.

7. Bausatz nach Anspruch 6, das des Weiteren eine Befestigungseinrichtung (194) umfasst, die zum Befestigen der ersten Sonde (190) oder der zweiten Sonde (190) an einem Schädel eines Empfängers ausgeführt ist.

8. Bausatz nach einem der Ansprüche 6 oder 7, der des Weiteren umfasst:
einen Puffer (406), der mit dem länglichen Träger (142) an einer Position gekoppelt ist, durch die verhindert wird, dass der längliche Träger (142) um mehr als eine vorgegebene Strecke vorgerückt wird.

9. Bausatz nach einem der Ansprüche 6, 7 oder 8,
wobei der Bausatz (200) sowohl die erste Sonde (190) als auch die zweite Sonde (190) einschließt;
der Bausatz (200) des Weiteren einen Positionierroboter (210) einschließt;
der Bausatz (200) des Weiteren einen Mikropositionierer (212) einschließt;
der Bausatz (200) des Weiteren einen Dichtstoff (214) oder einen Stopfen (216) einschließt, der zum Abdichten einer Öffnung eines Lumens (402) des länglichen Trägers (142) ausgeführt ist;
wobei die erste Sonde (190) oder die zweite Sonde (190) aus Metall, Glas oder Polymer aufgebaut ist;
wobei die erste Sonde (190) oder die zweite Sonde (190) von einem Anschlag (314) des länglichen Trägers (142) gelöst werden kann;
wobei die erste Sonde (190) oder die zweite Sonde (190) steif ist;
wobei die erste Sonde (190) oder die zweite Sonde (190) verformbar ist;
wobei die erste Sonde (190), die zweite Sonde (190) oder der längliche Träger (142) Formgedächtnis-Eigenschaften aufweist;
wobei die erste Sonde (190), die zweite Sonde (190) oder der längliche Träger (142) Formgedächtnis-Eigenschaften aufweist, die durch Temperatur aktiviert werden;
wobei die erste Sonde (190), die zweite Sonde (190) oder der längliche Träger (142) Formgedächtnis-Eigenschaften aufweist, die durch elektrischen Strom aktiviert werden; oder
wobei die erste Sonde (190), die zweite Sonde (190) oder der längliche Träger (142) ein Polymer mit Formgedächtnis-Eigenschaften aufweist, die durch elektrischen Strom aktiviert werden.

10. Bausatz nach einem der Ansprüche 6 - 9, wobei der Bausatz so ausgeführt ist, dass er über die Sonde die vestibuläre Elektrodenanordnung (140) zu der vestibulären Behandlungs-Zielposition (10) in dem Empfänger führt und dabei eine Steigbügel-Fußplatte des Empfängers als einen Drehpunkt einsetzt (544), der Feinsteuerung eines distalen Abschnitts der Sonde (190) ermöglicht.

11. Bausatz nach Anspruch 10,
wobei das Führen (530) einschließt:
Beziehen (560) elektrophysiologischer Rückmeldung bezüglich einer Position der vestibulären Elektrodenanordnung (140); sowie
Modifizieren der Position der vestibulären Elektrodenanordnung auf Basis der bezogenen elektrophysiologischen Rückmeldung.

12. Bausatz nach Anspruch 11, wobei Führen (530) der vestibulären Elektrodenanordnung (140) zu der vestibulären Behandlungs-Zielposition (10) in dem Empfänger des Weiteren einschließt:
Beziehen (552) eines Röntgenbildes, das eine Position eines radioopaken Markers (312) der vestibulären Elektrodenanordnung (140) relativ zu der vestibulären Behandlungs-Zielposition (10) zeigt.

13. Bausatz nach Anspruch 11 oder 12, wobei Beziehen der elektrophysiologischen Rückmeldung einschließt:
Erzeugen (562) eines Stimulus unter Verwendung wenigstens einer vestibulären Elektrode (144) der vestibulären Elektrodenanordnung (140); sowie Messen (564) einer Nerven- oder Muskelreaktion auf den erzeugten Stimulus.

14. Bausatz nach einem der Ansprüche 11 - 13, der des Weiteren Einrichtungen umfasst zum:
Überwachen (566) von Hören des Empfängers unter Einsatz von Elektrocochleographie, und/oder
Erzeugen (547) einer sichtbaren Anzeige oder einer hörbaren Anzeige der Position, und/oder
Erzeugen (548) der hörbaren Anzeige der Position über eine Änderung von Höhe eines Tons.

15. Bausatz nach einem der Ansprüche 11 - 14,
wobei das Führen (530) wenigstens teilweise von einem Positionierroboter (210) durchgeführt wird.

## Revendications

1. Appareil comprenant :
un support allongé (142) configuré pour être implanté près d'un organe vestibulaire d'un receveur ;
au moins une électrode vestibulaire (144) accouplée au support allongé (142) ; et
un faisceau de fibres optiques (220) d'une ou plusieurs fibres optiques (222),
**caractérisé en ce que**
le faisceau de fibres optiques (220) est accouplé au support allongé (142) de sorte qu'une implantation du support allongé (142) peut être surveillée par l'intermédiaire du faisceau de fibres optiques (220) ; et
que le faisceau de fibres optiques (220) est amovible du support allongé (142).

2. Appareil selon la revendication 1, dans lequel le support allongé (142) définit une rainure (350) au moins partiellement le long d'un côté du support allongé (142).

3. Appareil selon la revendication 2, dans lequel le support allongé (142) comprend une ou plusieurs travées (302) par-dessus la rainure (350) ; et
dans lequel les une ou plusieurs travées (302) sont configurées pour retenir le faisceau de fibres optiques (220) dans la rainure (350).

4. Appareil selon l'une quelconque des revendications 1, 2, ou 3, dans lequel un ou plusieurs composants de l'appareil sont radiopaques.

5. Appareil selon l'une quelconque des revendications 1, 2, ou 3, dans lequel le support allongé (142) définit un loquet (314) configuré pour recevoir un stylet (190) pour guider le support allongé (142) durant l'insertion du support allongé (142).

6. Kit comprenant :
un stimulateur vestibulaire implantable (110) ayant un logement biocompatible (111) ; et
l'appareil selon la revendication 5, dans lequel le support allongé (142) est configuré pour être accouplé, ou est accouplé, au stimulateur vestibulaire implantable (110) ;
dans lequel le stylet est n'importe lequel ou les deux parmi : un premier stylet (190) ayant une portion droite proche d'une première extrémité distale de stylet du premier stylet ; et un second stylet (190) ayant une portion incurvée proche d'une seconde extrémité distale de stylet du second stylet.

7. Kit selon la revendication 6, comprenant en outre : une attache (194) configurée pour fixer solidement le premier stylet (190) ou le second stylet (190) à un crâne d'un receveur.

8. Kit selon l'une quelconque des revendications 6 ou 7, comprenant en outre :
un arrêt (406) accouplé au support allongé (142) au niveau d'une localisation configuré pour résister au support allongé (142) qui est avancé de plus d'une distance prédéterminée.

9. Kit selon l'une quelconque des revendications 6, 7, ou 8,
dans lequel le kit (200) inclut à la fois le premier stylet (190) et le second stylet (190) ;
dans lequel le kit (200) inclut en outre un dispositif de positionnement robotique (210) ;
dans lequel le kit (200) inclut en outre un micropositionneur (212) ;
dans lequel le kit (200) inclut en outre un agent d'étanchéité (214) ou un bouchon (216) configuré pour sceller une ouverture d'une lumière (402) du support allongé (142) ;
dans lequel le premier stylet (190) ou le second stylet (190) est construit à partir d'un métal, d'un verre, ou d'un polymère ;
dans lequel le premier stylet (190) ou le second stylet (190) peut être retiré d'un loquet (314) du support allongé (142) ;
dans lequel le premier stylet (190) ou le second stylet (190) est rigide ;
dans lequel le premier stylet (190) ou le second stylet (190) est malléable ;
dans lequel le premier stylet (190), le second stylet (190) ou le support allongé (142) présentent des caractéristiques de mémorisation de forme ;
dans lequel le premier stylet (190), le second stylet (190), ou le support allongé (142) présentent des caractéristiques de mémorisation de forme configurées pour être activées par la température ;
dans lequel le premier stylet (190), le second stylet (190), ou le support allongé (142) présentent des caractéristiques de mémorisation de forme configurées pour être activées par un courant électrique ; ou
dans lequel le premier stylet (190), le second stylet (190), ou le support allongé (142) incluent un polymère ayant des caractéristiques de mémorisation de forme configurées pour être activées par un courant électrique.

10. Kit selon l'une quelconque des revendications 6 à 9, dans lequel le kit est configuré pour guider, par l'intermédiaire du stylet, la rangée d'électrodes vestibulaires (140) vers la localisation de traitement vestibulaire cible (10) chez le receveur en utilisant (544) une plate-forme de l'étrier du receveur comme pivot pour permettre un contrôle fin d'une portion distale du stylet (190).

11. Kit selon la revendication 10,
dans lequel le guidage (530) inclut :
l'obtention (560) d'une rétroaction électrophysiologique concernant une position de la rangée d'électrodes vestibulaires (140) ; et
une modification de la position de la rangée d'électrodes vestibulaires sur la base de la rétroaction électrophysiologique obtenue.

12. Kit selon la revendication 11, dans lequel le guidage (530) de la rangée d'électrodes vestibulaires (140) vers la localisation du traitement vestibulaire cible (10) chez le receveur inclut en outre l'étape consistant à :
obtenir (552) une image fluoroscopique montrant une localisation d'un marqueur radiopaque (312) de la rangée d'électrodes vestibulaires (140) par rapport à la localisation du traitement vestibulaire cible (10).

13. Kit selon la revendication 11 ou 12, dans lequel l'obtention de la rétroaction électrophysiologique inclut l'étape consistant à :
fournir (562) un stimulus utilisant au moins une électrode vestibulaire (144) de la rangée d'électrodes vestibulaires (140) ; et à mesurer (564) une réponse neuronale ou myogénique au stimulus fourni.

14. Kit selon l'une quelconque des revendications 11 à 13, comprenant en outre des moyens consistant à :
surveiller (566) une audition du receveur en utilisant l'électrocochléographie, et/ou
à fournir (547) une indication visuelle ou une indication audible de la position, et/ou
à fournir (548) l'indication audible de la position par l'intermédiaire d'un changement d'un niveau de tonalité.

15. Kit selon l'une quelconque des revendications 11 à 14,
dans lequel le guidage (530) est effectué au moins en partie par un dispositif de positionnement robotique (210).
